# FASCICULE DE BREVET EUROPEEN

(11) **EP 1 487 466 B1**
(45) Date de publication et mention de la délivrance du brevet: **09.01.2008**
(21) Numéro de dépôt: 03725310.1
(22) Date de dépôt: 21.03.2003
(51) Int. Cl.: A61K 35/74, A23L 1/03, A61P 1/00, A61P 29/00

(54) **UTILISATION DE LACTOBACILLUS FARCIMINIS POUR LA PREVENTION OU LE TRAITEMENT DE PATHOLOGIES DIGESTIVES**
VERWENDUNG VON LACTOBACILLUS FARCIMINIS ZUR PROPHYLAXE ODER BEHANDLUNG VON VERDAUUNGSERKRANKUNGEN
USE OF LACTOBACILLUS FARCIMINIS FOR THE PREVENTION OR THE TREATMENT OF DIGESTIVE PATHOLOGIES

(30) Priorité: 28.03.2002 FR 0203891
(43) Date de publication de la demande: 22.12.2004
(73) Titulaire: INSTITUT NATIONAL DE LA RECHERCHE AGRONOMIQUE (INRA), 75007 Paris Cédex 07 (FR)
(72) Inventeur: FIORAMONTI, Jean, F-31120 ROQUETTES (FR); BUENO, Lionel, F-31840 AUSSONNE (FR); THEODOROU, Vassilia, F-31120 PORTET-SUR-GARONNE (FR); LAMINE, Florence, F-31100 TOULOUSE (FR)
(74) Mandataire: Vialle-Presles, Marie José
(86) Numéro de dépôt international: PCT/FR2003/000903
(87) Numéro de publication internationale: WO 2003/082307

(56) Documents cités:
- WO-A-00/28943
- WO-A-98/27991
- WO-A-98/42200
- G. WOLF ET AL.: "Heme-dependent and heme-independent nitrite reduction by lactic acid bacteria results in different N-containing products." INTERNATIONAL JOURNAL OF FOOD MICROBIOLOGY, vol. 10, no. 3/4, 1990, pages 323-329, XP008012874 Amsterdam, NL cité dans la demande
- D.-M. MCCAFFERTY ET AL.: "Inducible nitric oxide synthase plays a crucial role in resolving intestinal inflammation" GASTROENTEROLOGY, vol. 112, no. 3, 1997, pages 1022-1027, XP002228823
- R. KORHONEN ET AL.: "Induction of nitric oxide synthesis by probiotic Lactobacillus rhamnosus GG in J774 macrophages and human T84 intestinal epithelial cells." INFLAMMATION, vol. 25, no. 4, août 2001 (2001-08), pages 223-232, XP008012977 cité dans la demande

## Description

La présente invention concerne l'utilisation de la bactérie lactique *Lactobacillus farciminis* pour la prévention ou le traitement de pathologies digestives, notamment fonctionnelles et/ou inflammatoires.

De nombreuses pathologies du tube digestif, et en particulier de l'intestin, impliquent à un degré plus ou moins important, des phénomènes inflammatoires. Parmi ces pathologies, on citera notamment :
- les maladies inflammatoires chroniques intestinales, qui englobent principalement la maladie de Crohn et la rectocolite hémorragique constituent une pathologie de prévalence faible, mais toutefois en augmentation. Ces maladies, très invalidantes, sont caractérisées par des poussées inflammatoires de gravité variable avec des phases de rémission parfois prolongées. La thérapeutique actuelle est principalement basée sur l'administration de corticoïdes, de 5-ASA (acide 5-amino-salicylique), la chirurgie dans les cas les plus graves. Des traitements avec certaines cytokines ont été proposés, mais ils sont encore au stade expérimental, et demeurent onéreux ;
- les troubles fonctionnels digestifs (TFD), qui constituent une pathologie à faible morbidité mais de très forte prévalence. La douleur viscérale est le principal symptôme, mais d'autres symptômes digestifs (diarrhée, constipation ou alternance des deux) ou extra digestifs (fatigue) sont souvent associés. Sa physiopathologie reste imprécise (altérations de la motricité gastro-intestinale, implications de facteurs psychosociaux, séquelles d'inflammation digestive ou de chirurgie), mais l'hypersensibilité douloureuse à la distension de la paroi digestive est une caractéristique principale de cette pathologie. L'origine de cette hypersensibilité n'est pas connue bien qu'une origine inflammatoire ait été supposée du fait de la prolongation des TFD pendant plusieurs mois chez une forte proportion de patients atteints d'une gastroentérite aiguë, et par la mise en évidence de séquelles inflammatoires (hyperplasie des mastocytes) dans des muqueuses digestives de patients présentant des TFD.

Différentes équipes ont rapporté l'efficacité de microorganismes probiotiques dans le cadre du traitement de ces pathologies du tube digestif. On regroupe sous l'appellation « probiotiques », des microorganismes vivants de différentes familles, genres et espèces, qui, lorsqu'ils sont ingérés en quantité suffisante, exercent un effet positif sur la santé, allant au-delà des effets nutritionnels traditionnels.

Des études montrant l'efficacité de probiotiques dans le traitement d'inflammations digestives expérimentales ont ainsi été réalisées avec *Lactobacillus reuteri* (FABIA et al., Scand. J. Gastroenterol., 28, 155-162, 1993 ; HOLMA et al., Scand. J. Gastroenterol., 36, 630-635, 2001) et *Lactobacillus plantarum* (MAO et al., Gastroenterology, 111, 334-344, 1996). Récemment, une étude chez l'homme a montré l'efficacité de *Saccharomyces boulardii* dans la prévention de la récidive de la maladie de Crohn (GUSLANDI et al., Dig. Dis. Sci., 45, 1462-1464, 2000). D'autres probiotiques ont également montré une efficacité dans le traitement de la rectocolite hémorragique : *Escherichia coli Nissle* (KRUIS et al., Aliment. Pharmacol. Ther., 11, 853-858, 1997 ; REMBACKEN et al., Lancet, 354, 635-639, 1999), une association de souches de *Bifidobacterium, Lactobacillus* et *Streptococccus* (VENTURI et al., Aliment. Pharmacol. Ther., 13, 1103-1108, 1999) et une association *Bifidobacterium bifidum, Bidobacterium breve, Lactobacillus acidophilus* (ISHIKAWA et al., Gastroenterology, 118, 4171, 2000).

En ce qui concerne les troubles fonctionnels digestifs, et le syndrome de l'intestin irritable, les résultats sont moins probants : une étude a montré que *Saccharomyces boulardii* réduisait la diarrhée associée aux troubles fonctionnels digestifs mais n'affectait pas les autres symptômes (MAUPAS et al., Méd. Chir. Dig., 12, 77-79, 1983), et une autre étude rapporte l'inefficacité d'une association de souches de *Lactobacillus* et *d'Escherichia coli* dans le traitement de la dyspepsie non ulcéreuse (HENTSCHEL et al., Gastroenterology, 112 Suppl 1, A146, 1997) ; des effets positifs de *Lactobacillus plantarum* sur plusieurs symptômes du syndrome de l'intestin irritable (flatulence, douleur abdominale) ont été observés, mais ces effets sont similaires à ceux d'un placebo pour le critère de la douleur abdominale (NOBAEK et al., Am. J. Gastroenterol., 95, 1231-1238, 2000) ; une autre étude portant sur trois des symptômes du syndrome de l'intestin irritable (douleur, « urgence toilette », ballonnements) rapporte l'absence d'effet de *Lactobacillus casei* CG (O' SULLIVAN et al., Dig. Liver Dis., 32, 294-301, 2000).

Il apparaît donc que l'utilisation de microorganismes probiotiques pour le traitement de pathologies inflammatoires du tube digestif constitue une approche prometteuse, mais dont l'efficacité apparaît variable, à la fois selon l'espèce de micro-organisme utilisé, et selon la pathologie ou le symptôme pathologique concerné. Il est donc souhaitable d'identifier d'autres microorganismes utilisables dans ce but, afin d'élargir la gamme des possibilités thérapeutiques.

Les Inventeurs ont maintenant découvert que des bactéries lactiques du genre *farciminis* de l'espèce *Lactobacillus* étaient actives *in vivo* sur l'inflammation du tube digestif, et notamment du colon, ainsi que sur la douleur viscérale. Les Inventeurs ont constaté que l'activité anti-inflammatoire de *Lactobacillus farciminis* était due à la production *in situ* dans la lumière digestive de monoxyde d'azote (NO) par cette bactérie.

*Lactobacillus farciminis* appartient au groupe I (homofermentaire stricte) de l'espèce *Lactobacillus.* Elle est fréquemment rencontrée dans différents produits alimentaires tels que les produits carnés, notamment les saucisses, et le levain de panification (DE ROISSARD et LUQUET, Bactéries lactiques, Volume I : Aspects fondamentaux et technologiques, Lorica, 1998). La production de monoxyde d'azote en culture par *Lactobacillus farciminis* a été rapportée par WOLF et al., Int. J. Food Microbiol., 10, 323-329, 1990.

Un rôle potentiel du monoxyde d'azote dans la régulation des fonctions digestives et/ou la protection de la muqueuse digestive a été suggéré par différentes observations. On sait que certaines cellules de l'épithélium intestinal peuvent produire du monoxyde d'azote, après induction par certaines cytokines pro-inflammatoires et/ou par les toxines lipopolysaccharidiques (LPS) de bactéries entéroinvasives (WITTHOFT et al., Am. J. Physiol., 275, G564-571, 1998). Ce monoxyde d'azote endogène, participerait, par ses propriétés antimicrobiennes, à la défense contre les microorganismes pathogènes. Il participerait également, lorsqu'il est produit en faibles quantités, à la protection de la muqueuse intestinale. Cependant, en quantités plus importantes, il contribuerait à l'instauration et à l'entretien d'un état inflammatoire chronique (ALICAN et KUBES, Am. J. Physiol. 270, G225-237, 1996 ; TEPPERMAN et al., J. Pharmacol. Exp. Ther., 271, 1477-1482, 1994).

En ce qui concerne le monoxyde d'azote exogène (c'est à dire provenant de l'alimentation ou d'une administration médicamenteuse), les effets observés sont également contradictoires ; un effet protecteur transitoire de donneurs de NO vis-à-vis de lésions induites par l'éthanol sur la muqueuse gastrique a été observé (MAC NAUGHTON et al., Life Sci., 45, 1869-1876, 1989), de même qu'un effet protecteur vis-à-vis de lésions induites par l'acide chlorhydrique (KITAGAWA et al., J. Pharmacol. Exp. Ther., 253, 1133-1137, 1990);. D'autres travaux ont montré que la perfusion intra-artérielle locale de donneurs de NO peut induire des effets variables vis-à-vis d'altérations hémorragiques de la muqueuse gastrique : un effet protecteur, une absence d'effet ou un effet délétère peuvent être observés, selon la nature du donneur de NO et la dose utilisée (LOPEZ-BELMONTE et al., Br. J. Pharmacol., 108, 73-78, 1993).

On connaît actuellement quelques microorganismes probiotiques dont les effets apparaissent dus, au moins en partie, à une influence sur la production de NO endogène. La Demande PCT WO 00/28943 montre que certaines souches de bactéries lactiques, et notamment de *Lactobacillus casei,* peuvent avoir une action anti-inflammatoire en augmentant la production de monoxyde d'azote par les entérocytes activés par des cytokines pro-inflammatoires, et en diminuant au contraire la production de monoxyde d'azote par les entérocytes activés par des cytokines pro-inflammatoires et des lipopolysaccharides bactériens. KORHONEN et al. (Inflammation, 25, 223-232, 2001) montrent que la souche GG de *Lactobacillus rhamnosus,* qui est active sur des diarrhées virales ou induites par les antibiotiques, peut augmenter la production de monoxyde d'azote par des cellules épithéliales intestinales ou des macrophages activés par des cytokines pro-inflammatoires, et indiquent que cet effet sur la production de NO pourrait être impliqué dans l'activité de *Lactobacillus rhamnosus.*

Les éventuels effets probiotiques de microorganismes directement producteurs de monoxyde d'azote n'ont été que très peu étudiés. La Demande PCT WO 98/27991 propose l'utilisation de bactéries du genre *Propionobacter,* productrices de monoxyde d'azote pour l'obtention d'une composition produisant des quantités physiologiquement significatives de NO dans le tube digestif, et rapporte un effet de cette composition sur la motricité intestinale. Ce document mentionne également *Lactobacillus farciminis,* mais pour en exclure l'utilisation, sur la base d'essais expérimentaux en culture d'où il est conclu que la quantité de NO produite par *L. farciminis* est trop faible pour être significative.

Contrairement à ce qui était indiqué dans la Demande PCT WO 98/27991, les Inventeurs ont maintenant établi que *L. farciminis* produit, dans le tube digestif, une quantité de monoxyde d'azote lui permettant d'exercer un effet thérapeutique, notamment un effet anti-inflammatoire, et un effet sur la douleur liée à la distension viscérale.

La présente invention a pour objet l'utilisation d'une bactérie lactique de l'espèce *Lactobacillus farciminis* pour l'obtention d'une composition destinée au traitement ou à la prévention de pathologies du tube digestif.

La souche type de *L. farciminis* qui a été utilisée dans le cadre des expérimentations qui ont conduit à la présente invention est connue en elle même et accessible dans différentes collections ; elle est par exemple référencée sous les numéros d'accès suivants : CIP-103136T, ATCC 29644, DSM 20184, JCM 1097, LMG 9200, NCDO 2330, NCIB 11717, IMET 11462. On peut également utiliser, pour la mise en oeuvre de la présente invention, des souches de *L. farciminis* isolées à partir de produits alimentaires contenant cette bactérie.

Selon un mode de mise en oeuvre préféré de la présente invention, ladite composition est destinée au traitement de pathologies inflammatoires aiguës ou chroniques du tube digestif, et notamment de l'intestin.

Selon un autre mode de mise en oeuvre préféré de la présente invention, ladite composition est destinée au traitement ou à la prévention de la douleur viscérale.

A titre d'exemples de pathologies pour le traitement desquelles la présente invention peut être mise en oeuvre, on citera les colites, les entérites, la maladie de Crohn, la rectocolite hémorragique, les troubles fonctionnels digestifs (syndrome de l'intestin irritable et dyspepsie non-ulcéreuse), etc.

De préférence, ladite composition est destinée à l'administration par voie orale.

Elle peut comprendre une ou plusieurs souches de *L. farciminis,* dans n'importe quelle formulation permettant de conserver ces bactéries viables, pendant les différentes étapes de leur conditionnement et de leur stockage, et après leur ingestion, jusqu'à leur site d'action dans le tube digestif.

Elle peut également comprendre éventuellement d'autres bactéries lactiques, possédant ou non des propriétés probiotiques, par exemple à des bactéries telles que les lactobacilles, les lactocoques, les streptocoques et les bifidobactéries, et/ou d'autres microorganismes probiotiques, tels que des levures.

Dans le cadre de la présente invention, les compositions comprenant *L. farciminis* peuvent être administrées sous forme d'aliments. Il peut s'agir par exemple de produits fermentés tels que des produits laitiers ; dans ce cas, *L. farciminis* peut faire partie du ferment mis en oeuvre pour l'obtention de ces produits, ou bien être ajoutée à ceux-ci après fermentation. Elles peuvent également être administrées sous forme de compléments alimentaires à incorporer dans l'alimentation, ou à ingérer directement. Avantageusement, elles peuvent être conditionnées sous forme de doses individuelles renfermant la quantité de *L. farciminis* souhaitée.

Pour la mise en oeuvre de la présente invention, *L. farciminis* sera de préférence administré à raison d'au moins 10⁶ UFC (unités formant colonie)/jour, avantageusement au moins 10⁸ UFC/jour, et de manière tout a fait préférée au moins 10¹⁰ UFC/jour, en une ou plusieurs prises.

La présente invention sera mieux comprise à l'aide du complément de description qui va suivre, qui se réfère à des exemples non-limitatifs illustrant les propriétés d'une souche de *Lactobacillus farciminis* vis à vis d'une inflammation colique et de la douleur viscérale à la distension.

### EXEMPLE 1 : EFFET D'UN TRAITEMENT PAR LACTOBACILLUS FARCIMINIS SUR UNE INFLAMMATION COLIQUE INDUITE PAR LE TNBS/ETHANOL : ROLE DU MONOXYDE D'AZOTE (NO)

Une inflammation colique (ou colite) peut être induite expérimentalement par l'acide trinitro-benzènesulfonique (TNBS), qui constitue le modèle d'inflammation expérimentale colique le plus courant et le mieux validé (MORRIS et al., Gastroenterology, 96, 795-803, 1989).

Cette colite se caractérise par une augmentation de l'activité myéloperoxydase (MPO), un marqueur du degré d'infiltration de polynucléaires neutrophiles dans la muqueuse colique, ainsi que par l'augmentation du score lésionnel macroscopique (SLM) prenant en compte la gravité et l'étendue des lésions macroscopiques apparues, la présence et la gravité des adhérences et la présence ou non de diarrhée dans le côlon.

Les effets d'un traitement par un donneur de monoxyde d'azote, le nitroprussiate de sodium (SNP), ou par *L. farciminis* sur une colite induite par le TNBS chez le rat ont été comparés. Par ailleurs, le rôle effectif du monoxyde d'azote exogène dans ces effets, a été évalué en utilisant un piégeur de NO, l'hémoglobine (Hb).

Pour l'étude des effets du SNP, 7 lots de rats WISTAR de 200-250 grammes sont équipés, sous anesthésie, d'un cathéter intracolique (+ 2cm de la jonction caeco-colique) extériorisé au niveau de la région dorso-scapulaire. A J+5, les rats reçoivent une instillation intracolique de 80 mg/kg/jour de TNBS/éthanol (lots 4-7) ou d'une solution NaCl 0,9% (lots 1-3). 4 heures après l'instillation, les rats sont perfusés à un débit de 250 µl/heure avec 1 mg/kg/jour de SNP (lots 2 et 5), ou 200 mg/kg/jour d'Hb (lots 3 et 7), ou un mélange SNP + Hb (lot 6), ou une solution NaCl 0,9% (lots 1 et 4), pendant 4 jours.

Pour l'étude des effets de *L. farciminis,* 5 lots de 10 rats mâles WISTAR de 200-250 grammes reçoivent par voie orale 10¹² UFC/jour de *L. farciminis* (lots 2, 4, 5) ou une solution NaCl 0,9% (lots 1, 3), pendant 19 jours. A J+10, les rats sont équipés d'un cathéter intracolique tel que précédemment décrit. A J+15, les rats reçoivent par voie intracolique 80 mg/kg de TNBS/éthanol (lots 3-5) ou une solution NaCl 0,9% (lots 1, 2). 4 heures après l'instillation, les rats reçoivent une perfusion intracolique d'Hb, à 200 mg/kg/jour (lot 5) ou d'une solution NaCl 0,9% (lots 1-4), pendant 4 jours.

A J+19, tous les rats sont sacrifiés et l'intensité de l'inflammation de la paroi du côlon est caractérisée par l'activité myéloperoxydase (MPO) et le score lésionnel macroscopique (SLM), sur des échantillons coliques isolés.

### I- Activité myéloperoxydase (MPO)

L'activité myéloperoxydase (MPO) est déterminée, sur des échantillons coliques isolés, selon le protocole décrit par BRADLEY et al., (J. Invest. Dermatol., 78, 206-209, 1982).

Ce protocole peut être résumé comme suit :

Les segments de côlon (1 cm de long) sont immergés dans du tampon phosphate (50 mM, pH 6). Après lyse mécanique sur glace à l'aide d'un homogénéiseur POLYTRON, 3 cycles de congélation (azote liquide, 1 min) et de décongélation (bain-marie, 37°C, 10 min) sont réalisés. Après centrifugation, (10000 rpm, 15 min, 4°C), le culot est repris dans du bromure d'hexadécyl triméthylammonium (HTAB) 0,5%. Les échantillons sont ensuite soniqués avant de subir une nouvelle centrifugation. Le surnageant est récupéré en vue des dosages de l'activité MPO et des protéines totales.

L'activité MPO est déterminée par spectrophotométrie. L'échantillon est mis en présence de tampon phosphate contenant du dihydrochlorure de O-dianisidine (0,167 mg/ml) et du peroxyde d'hydrogène 0,0005%. Les changements d'absorbance (450 nm, 25°C) ont été déterminés par spectrophotométrie cinétique de 2 minutes, d'après l'équation bilan de la réaction enzymatique catalysée par la MPO : H₂O₂ + Cl⁻ = H₂O + HOCl (coloration orangée), et ramenés en unités MPO.

Une unité d'activité MPO est définie comme la quantité de MPO dégradant 1 µmol de peroxyde d'hydrogène par minute par millilitre à 25°C. La concentration en protéines (grammes/ml) est déterminée à l'aide d'un kit commercial (Detergent Compatible Assay, Bio Rad, Ivry/Seine, France). L'activité MPO est exprimée sous forme d'unités MPO par gramme de protéines (U MPO/g de protéines).

### 1) Traitement par le SNP

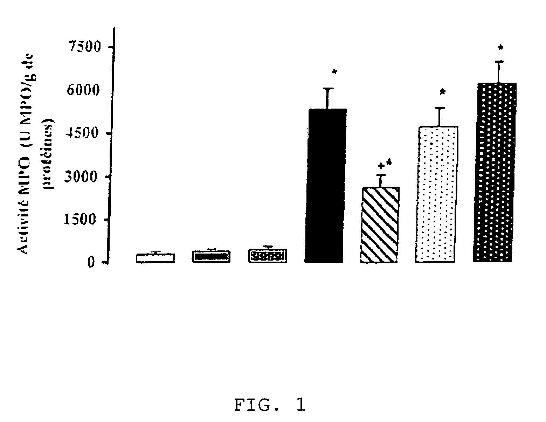

Les résultats sont présentés dans la Figure 1.

Légende de la Figure 1 :
En abscisse
□ = rats non traités (lot 1)
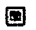 = rats traités par SNP (lot 2)
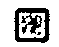 = rats traités par Hb (lot 3)
■ = rats traités par TNBS/éthanol (lot 4)
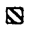 = rats traités par TNBS/éthanol + SNP (lot 5)
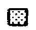 = rats traités par TNBS/éthanol + SNP + Hb (lot 6)
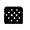 = rats traités par TNBS/éthanol + Hb (lot 7)
En ordonnée = activité MPO (U MPO/g de protéines)
* : significativement différente (P<0,01) du lot témoin (lot 1)
+ : significativement différente (P<0,01) du lot TNBS/éthanol (lot 4)

L'activité MPO de la paroi du côlon chez les rats contrôles (lot 1) et ceux traités par le SNP (lot 2) ou l'Hb (lot 3), en l'absence d'instillation de TNBS/éthanol est respectivement de 263±103 ; 351+88 ; 426±117 U MPO/g de protéines. Ces valeurs ne sont pas significativement différentes entre elles. L'instillation de TNBS/éthanol (lot 4) augmente de façon significative l'activité MPO par rapport aux rats contrôles (5346±714 U MPO/g de protéines). La perfusion de SNP chez les rats instillés au TNBS/éthanol (lot 5) diminue significativement l'activité MPO (2619±447 U MPO/g de protéines) par rapport aux rats ayant seulement reçu une instillation de TNBS/éthanol. La perfusion conjointe de SNP et d'Hb chez les rats instillés au TNBS/éthanol (lot 6) abolit la réduction de l'activité MPO induite par le SNP (4710±645 U MPO/g de protéines), aucune différence significative n'apparaissant par rapport aux rats instillés au TNBS/éthanol (lot 4 ; 5346±714 U MPO/g de protéines).

### 2) Traitement par L. farciminis

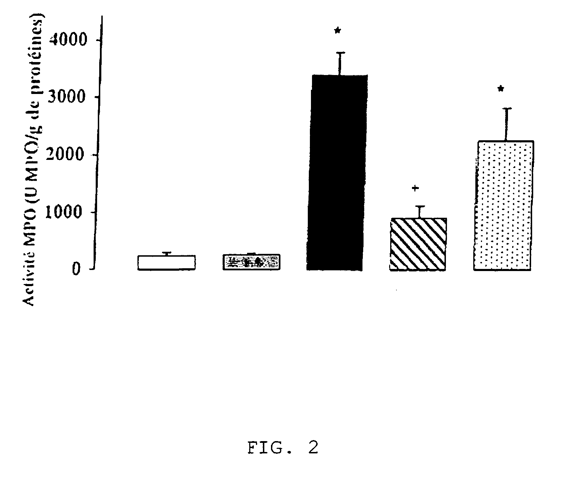

Les résultats sont illustrés par la Figure 2.

Légende de la Figure 2 :
En abscisse
□ = rats non traités (lot 1)
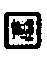 = rats traités par *L. farciminis* (lot 2)
■ = rats traités par TNBS/éthanol (lot 3)
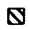 = rats traités par *L. farciminis* + TNBS/éthanol (lot 4)
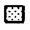 = rats traités par *L. farciminis* + TNBS/éthanol + Hb (lot 5)
En ordonnée = activité MPO (U MPO/g de protéines)
* : significativement différente (P<0,01) du lot témoin (lot 1)
+ : significativement différente (P<0,01) du lot TNBS/éthanol (lot 3)

Les rats contrôles non traités (lot 1) ont une activité MPO de 237±53 U MPO/g de protéines.

L'instillation de TNBS/éthanol induit une inflammation colique caractérisée par une augmentation de l'activité MPO (lot 3 ; 3900±395 U MPO/g de protéines). Le traitement par *L. farciminis* n'a pas d'effet sur l'activité MPO chez les rats instillés avec la solution saline (lot 2 ; 256±31 U MPO/g de protéines). Au contraire, chez les rats instillés au TNBS/éthanol, le traitement par *L. farciminis* diminue de façon significative l'activité MPO (lot 4 ; 905±211 U MPO/g de protéines). La perfusion d'hémoglobine abolit la réduction de l'inflammation par *L. farciminis* chez les rats instillés au TNBS/éthanol (lot5 ; 2246±566 U MPO/g de protéines).

### II- Score lésionnel macroscopique (SLM)

Le score lésionnel macroscopique (SLM) est déterminé sur des échantillons coliques isolés tel que décrit par WALLACE et al., (Gastroenterology, 102, 18-27, 1992) d'après la grille d'évaluation ci-dessous :

| Paramètre | Score |
|---|---|
| Ulcération | |
| Apparence normale | 0 |
| Hyperémie focale sans ulcération | 1 |
| Ulcération sans hyperémie ou épaississement de la muqueuse | 2 |
| Ulcération à 1 site inflammatoire | 3 |
| Ulcération à 2 sites inflammatoires ou plus | 4 |
| Plusieurs sites inflammatoires sur plus de 1 cm | 5 |
| Aire inflammatoire >2cm, score augmenté de 1 à chaque cm ulcéré | 6-10 |
| Adhésion | |
| Pas d'adhérence | 0 |
| Adhérence légère | 1 |
| Adhérence forte | 2 |
| Diarrhée | |
| Non | 0 |
| Oui | 1 |
| Score total | |

### 1) Traitement par le SNP

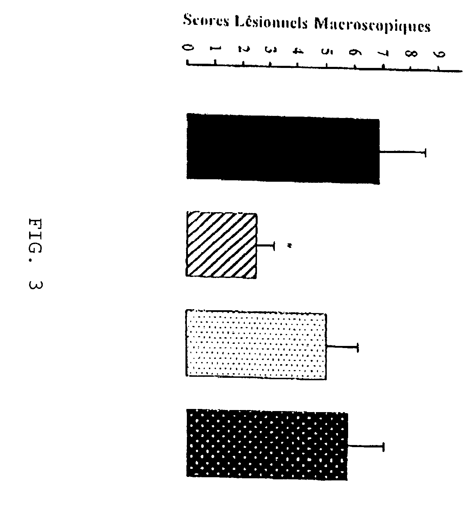

Les résultats sont illustrés par la Figure 3.

Légende de la Figure 3 :
En abscisse
■ = rats traités par TNBS/éthanol (lot 4)
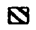 = rats traités par TNBS/éthanol + SNP (lot 5)
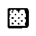 = rats traités par TNBS/éthanol + SNP + Hb (lot 6)
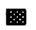 = rats traités par TNBS/éthanol + Hb (lot 7)
En ordonnée = scores lésionnels macroscopiques
* : significativement différent (P<0,01) du lot témoin (lots 1-3)

4 jours après l'instillation de TNBS/éthanol, la muqueuse colique se caractérise par une importante ulcération associée à une inflammation régulière et une épaisseur de paroi correspondant à un SLM de 6,9±1,7 (lot 4). Le traitement journalier par le SNP réduit de façon significative l'étendue de la lésion colique, diminuant le SLM à 2,5±0,6 (lot 5), alors que le traitement journalier avec le SNP + Hb et Hb seul n'a pas d'effet sur ce paramètre avec des SLM respectifs de 5,0±1,1 (lot 6) et 5,8±1,3 (lot 7).

### 2) Traitement par Lactobacillus farciminis

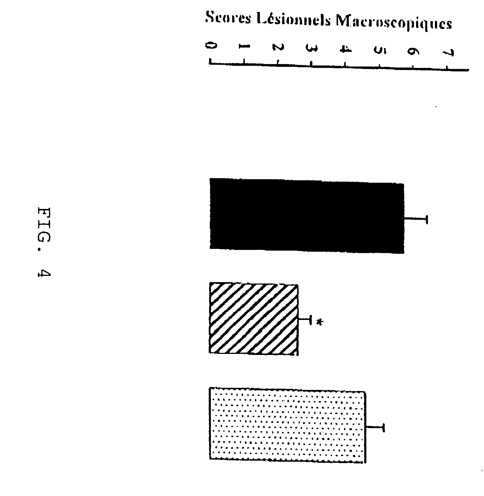

Les résultats sont illustrés par la Figure 4.

Légende de la Figure 4 :
En abscisse
■ = rats traités par TNBS/éthanol (lot 3)
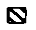 = rats traités par *L. farciminis* + TNBS/éthanol (lot 4)
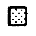 = rats traités par *L. farciminis* + TNBS/éthanol + Hb (lot 5)
En ordonnée = scores lésionnels macroscopiques
* : significativement différent (P<0,01) du lot témoin (lots 1 et 2)

Par rapport aux rats contrôles considérés comme dépourvus de lésions macroscopiques (lots 1 et 2), le TNBS/éthanol induit une inflammation colique caractérisée par des lésions macroscopiques (lot 3 ; 5,7±0,7). Chez les rats instillés au TNBS/éthanol, le traitement par *L. farciminis* réduit de façon très significative le score lésionnel (lot 4 ; 2,6±0,9). Cet effet n'apparaît plus lorsque le traitement par *L. farciminis* est accompagné d'une perfusion intracolique d'Hb (lot 5 ; 4,6±0,5).

### III- Conclusion

L'administration orale de *L. farciminis* réduit, de manière similaire à un traitement par le SNP, l'activité myéloperoxydase et le score lésionnel des rats traités par le TNBS/éthanol.

Ces effets anti-inflammatoires sont abolis par l'administration d'une perfusion intracolique d'hémoglobine, ce qui montre qu'ils mettent en jeu la production de NO.

### EXEMPLE 2 : EFFET CURATIF DE LACTOBACILLUS FARCIMINIS SUR UNE INFLAMMATION COLIQUE PAR LE TNBS/ETHANOL

Les effets d'un traitement curatif par *L. farciminis* débutant au moment de l'induction d'une l'inflammation colique par le TNBS/éthanol, ont été étudiés.

4 lots de 10 rats mâles WISTAR de 200-250 grammes sont équipés, sous anesthésie, d'un cathéter intracolique tel que décrit à l'Exemple 1.

A J+5, les rats reçoivent une instillation par voie intracolique de 80 mg/kg de TNBS/éthanol (lots 1 et 2) ou d'une solution NaCl 0,9% (lots 3 et 4).

Le traitement par *L. farciminis* débute 4 heures après l'induction de l'inflammation. Les rats reçoivent par voie orale 10¹² ufc/jour de *Lactobacillus farciminis* (lots 1 et 3) ou une solution NaCl 0,9% (lots 2 et 4), pendant 4 jours.

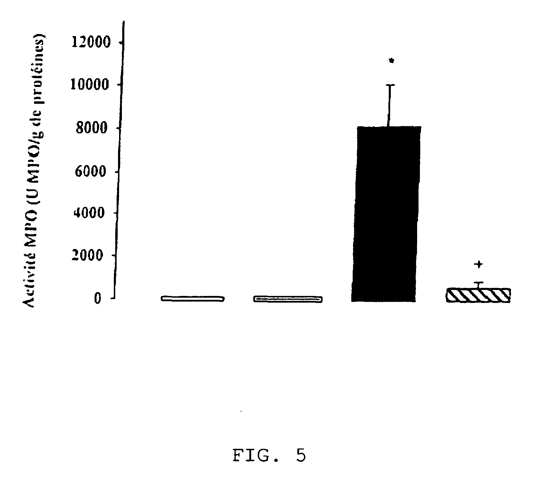

A J+4, les rats sont sacrifiés et l'intensité de l'inflammation de la paroi du côlon est caractérisée par l'activité myéloperoxydase (MPO), sur des échantillons coliques isolés, tel que décrit à l'Exemple 1. Les résultats sont présentés dans la Figure 5.

Légende de la Figure 5 :
En abscisse
□ = rats non traités (lot 4)
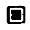 = rats traités par *L. farciminis* (lot 3)
■ = rats traités par TNBS/éthanol (lot 2)
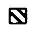 = rats traités par *L. farciminis* + TNBS/éthanol (lot 1)
En ordonnée = activité MPO (U MPO/g de protéines)
* : significativement différente (P<0,01) du lot témoin (lot 4)
+ : significativement différente (P<0,01) du lot TNBS/éthanol (lot 2)

L'activité myéloperoxydase est fortement augmentée chez les rats traités par le TNBS/éthanol (lot 2 ; 8184±1946 U MPO/g de protéines) par rapport aux rats contrôles non traités (lot 4 ; 76±16 U MPO/g de protéines). Le traitement par *L. farciminis* n'augmente pas l'activité MPO chez les rats instillés par la solution saline (lot 3 ; 128±48 U MPO/g de protéines). Au contraire, le traitement par *L. farciminis* des rats instillés au TNBS/éthanol (lot 1 ; 584±299 U MPO/g de protéines) réduit de façon très significative l'activité myéloperoxydase.

### EXEMPLE 3 : EFFET D'UN TRAITEMENT PAR LACTOBACILLUS FARCIMINIS SUR LA DOULEUR A LA DISTENSION COLORECTALE

Les effets d'un traitement par *L. farciminis* vis à vis de la douleur viscérale induite par distension colorectale ont été étudiés. Cette étude a été effectuée chez des rats sains (conditions basales) ou en condition d'hyperalgésie induite par une inflammation colique (conditions d'inflammation) ou un stress de contrainte (conditions de stress). La douleur à la distension se manifeste par l'augmentation du nombre de contractions des muscles abdominaux (MORTEAU et al., Dig. Dis. Sci., 39, 1239-1248, 1994).

Pendant 21 jours, 7 lots de 10 rats mâles WISTAR de 200-250 grammes reçoivent par voie orale 10¹² ufc/jour de *Lactobacillus farciminis* (lots 2, 4 et 7) ou une solution NaCl 0,9% (lots 1, 3, 5 et 6).

A J+7, les rats sont équipés, sous anesthésie, d'un cathéter intracolique (+ 2cm de la jonction caeco-colique) et 3 groupes de 3 électrodes en NiCr sont implantés de chaque côté du muscle oblique externe abdominal juste au-dessus du ligament inguinal. Le cathéter et les électrodes sont accessibles de l'extérieur au niveau de la région dorso-scapulaire et protégés par un tube en verre fixé à la peau.

### I- Effet d'un traitement par L. farciminis en conditions basales

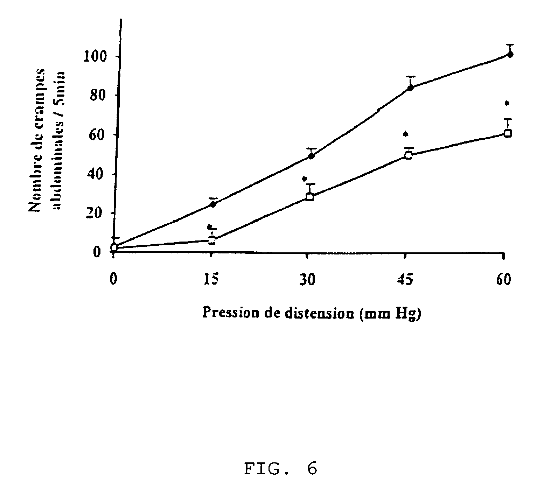

A J+15, une distension colorectale est réalisée à l'aide d'un ballon inséré par voie rectale, à 5 cm de l'anus et fixé à la queue de l'animal. Le ballon est gonflé progressivement de 0 à 60 mm Hg, par étapes de 15 mm Hg, chaque étape durant 5 minutes. Les contractions du muscle abdominal sont enregistrées avec un électroencéphalographe pour visualiser la sensibilité viscérale. Les résultats sont illustrés par la Figure 6.

Légende de la Figure 6 :
En abscisse = pression de distension (mm Hg)
En ordonnée = nombre de crampes abdominales/5 min
* : significativement différent (P<0,05) du lot témoin (lot 1)
Lots de rats utilisés :
◆ = rats non traités (lot 1)
□ = rats traités par *L. farciminis* (lot 2)

La distension colorectale progressive augmente le nombre des contractions du muscle abdominal en fonction du volume de distension, que les rats aient été traités par *L. farciminis* (lot 2) ou non (lot 1). Cependant, quel que soit le volume de distension, le nombre des contractions du muscle abdominal est diminué chez les rats traités par *L. farciminis* (lot 2) par rapport aux rats non traités (lot 1).

### II- Effet d'un traitement par L. farciminis en conditions d'inflammation.

A J+17, les rats reçoivent l'instillation intracolique de 80 mg/kg de TNBS/éthanol (lots 3 et 4) ou une solution NaCl 0,9% (lot 1).

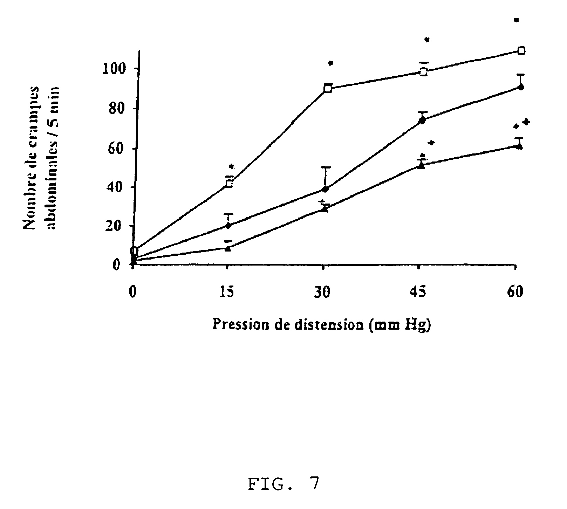

A J+21, soit 4 jours après l'instillation intracolique, une nouvelle session de distensions colorectales est réalisée comme décrit ci-dessus. Les contractions du muscle abdominal sont enregistrées avec un électroencéphalographe pour visualiser la sensibilité viscérale. Les résultats sont illustrés par la Figure 7.

Légende de la Figure 7 :
En abscisse = pression de distension (mm Hg)
En ordonnée = nombre de crampes abdominales/5 min
* : significativement différent (P<0,05) du lot témoin (lots 1 et 3)
Lots de rats utilisés :
◆ = rats non traités (lot 1)
□ = rats traités par TNBS/éthanol (lot 3)
▲ = rats traités par *L. farciminis* + TNBS/éthanol (lot 4)

Le nombre des contractions du muscle abdominal est augmenté de façon significative 4 jours après l'instillation de TNBS/éthanol chez les rats non traités par *L. farciminis* (lot 3) par rapport aux rats contrôles (lot 1). Le nombre des contractions du muscle abdominal est diminué de façon significative 4 jours après l'instillation de TNBS/éthanol chez les rats traités par *L. farciminis* (lot 4) par rapport aux rats ayant reçu le TNBS/éthanol mais non traités par *L. farciminis* (lot 3) et n'est pas significativement différent de celui des rats contrôles (lot 1).

### III- Effet d'un traitement par L. farciminis en conditions de stress

A J+15 les rats sont soumis à un stress de contrainte (lots 6 et 7). Sous anesthésie légère à l'éther, le thorax et les membres antérieurs des rats sont entourés de ruban adhésif de manière à limiter leurs mouvements. Ils sont maintenus dans cette position pendant 2 heures. Les animaux témoins n'ont été soumis qu'à l'anesthésie à l'éther (lot 5).

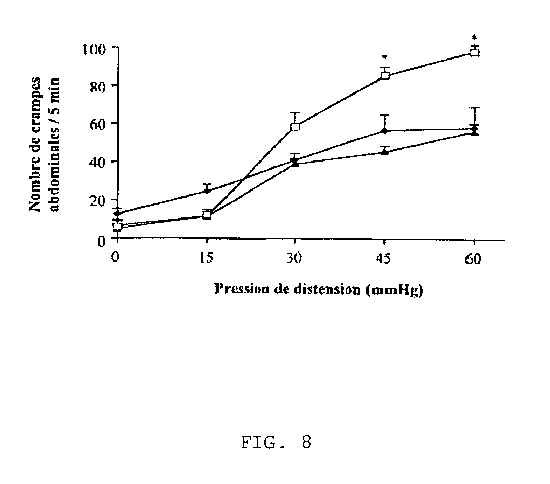

Vingt minutes après le stress de contrainte, une session de distensions colorectales est réalisée comme décrit ci-dessus. Les contractions du muscle abdominal sont enregistrées avec un électroencéphalographe pour visualiser la sensibilité viscérale. Les résultats sont illustrés par la Figure 8.

Légende de la Figure 8 :
En abscisse = pression de distension (mm Hg)
En ordonnée = nombre de crampes abdominales/5 min
* : significativement différent (P<0.05) du lot témoin (lot 5)
Lots de rats utilisés
**◆** = rats témoins non traités et non stressés (lot 5)
□ = rats non traité et stressés (lot 6)
▲ = rats traités par *L. farciminis* et stressés (lot 7)

Le nombre de contractions abdominales correspondant à des distensions colorectales de 45 et 60 mm Hg est augmenté de façon significative après stress chez les rats non traités par *L. farciminis* (lot 6) par rapport aux rats contrôles (lot 5). Pour les pressions de 45 et 60 mm Hg, le nombre de contractions abdominales chez les rats traités par *L. farciminis* et stressés (lot 7) est significativement diminué par rapport au animaux non traités et stressés (lot 6) et n'est pas significativement différent de celui des animaux contrôles non traités et non stressés (lot 5).

### IV- Conclusion

Ces résultats montrent qu'un traitement par *L. farciminis,* en conditions basales ou d'hyperalgésie induite par une inflammation colique ou un stress de contrainte, réduit le nombre de contractions des muscles abdominaux, indiquant une diminution de la douleur viscérale.

## Revendications

1. Utilisation de bactéries lactiques de l'espèce *Lactobacillus farciminis* pour l'obtention d'une composition destinée au traitement ou à la prévention d'une pathologie du tube digestif.

2. Utilisation selon la revendication 1, **caractérisée en ce que** ladite pathologie est une pathologie inflammatoire aiguë ou chronique de l'intestin.

3. Utilisation selon une quelconque des revendications 1 ou 2, **caractérisée en ce que** ladite pathologie se manifeste par des douleurs viscérales.

4. Utilisation selon une quelconque des revendications 1 à 3, **caractérisée en ce que** ladite composition est sous la forme d'un aliment ou d'un complément alimentaire.

5. Composition pour le traitement de pathologies du tube digestif, **caractérisée en ce qu'**elle comprend des bactéries lactiques de l'espèce *L. farciminis.*

## Claims

1. The use of a lactic acid bacterium of the species *Lactobacillus farciminis* for the production of a composition intended for the treatment or prevention of pathologies of the digestive tube.

2. The use as claimed in claim 1, **characterized in that** said pathology is an acute or chronic inflammatory pathology of the intestine.

3. The use as claimed in either of claims 1 and 2, **characterized in that** said pathology manifests itself by visceral pain.

4. The use as claimed in any one of claims 1 to 3, **characterized in that** said composition is in the form of a foodstuff or a dietary supplement.

5. A composition for the treatment of pathologies of the digestive tube, **characterized in that** it comprises lactic acid bacteria of the species *L. farciminis.*

## Patentansprüche

1. Verwendung von Milchsäurebakterien der Art *Lactobacillus farciminis* zur Herstellung einer Zusammensetzung, die zur Behandlung oder Verhinderung einer Erkrankung des Verdauungskanals bestimmt ist.

2. Verwendung nach Anspruch 1, **dadurch gekennzeichnet, dass** die Erkrankung eine akute oder chronische entzündliche Erkrankung des Intestinums ist.

3. Verwendung nach einem der Ansprüche 1 oder 2, **dadurch gekennzeichnet, dass** sich die Erkrankung durch viszerale Schmerzen manifestiert.

4. Verwendung nach einem der Ansprüche 1 bis 3, **dadurch gekennzeichnet, dass** die Zusammensetzung in Form eines Nahrungsmittels oder eines Nahrungsergänzungsmittels ist.

5. Zusammensetzung zur Behandlung von Erkrankungen des Verdauungskanals, **dadurch gekennzeichnet, dass** sie Milchsäurebakterien der Art *L. farciminis* umfasst.
